# EUROPÄISCHE PATENTSCHRIFT

(11) **EP 0 703 961 B1**
(45) Veröffentlichungstag und Bekanntmachung des Hinweises auf die Patenterteilung: **17.09.1997**
(21) Anmeldenummer: 94918860.1
(22) Anmeldetag: 09.06.1994
(51) Int. Cl.: C11D 1/66, C11D 1/83, C11D 3/20, C11D 17/00

(54) **FLÜSSIGKRISTALLINE WÄSSRIGE TENSIDZUBEREITUNG**
LIQUID-CRYSTALLINE AQUEOUS SURFACTANT PREPARATION
PREPARATIONS TENSIOACTIVES AQUEUSES A CRISTAUX LIQUIDES

(30) Priorität: 18.06.1993 DE 4320119
(43) Veröffentlichungstag der Anmeldung: 03.04.1996
(73) Patentinhaber: Henkel Kommanditgesellschaft auf Aktien, 40191 Düsseldorf (DE)
(72) Erfinder: NICKEL, Dieter, D-40699 Erkrath (DE); HOFFMANN, Rainer, D-40597 Düsseldorf (DE); WEUTHEN, Manfred, D-42697 Solingen (DE)
(86) Internationale Anmeldenummer: EP9401890
(87) Internationale Veröffentlichungsnummer: WO9500612

(56) Entgegenhaltungen:
- EP-A- 0 388 810
- US-A- 4 396 520

## Beschreibung

Die Erfindung betrifft niedrigkonzentrierte flüssigkristalline wäßrige Tensidzubereitungen, die Alkylglykoside und bestimmte langkettige Alkohole sowie gegebenenfalls synthetische Aniontenside enthalten, ein Verfahren zu ihrer Herstellung sowie die Verwendung derartiger Zubereitungen zur Herstellung von Wasch- oder Reinigungsmitteln.

Daß Alkylglykoside mit langkettigen Alkylgruppen zu den nichtionischen Tensiden gehören, ist seit langer Zeit bekannt. Ebenso weiß der Fachmann, wie zum Beispiel in A.M. Schwartz, J.W. Perry, Surface Active Agents, Vol. I, Interscience Publishers, 1949, Seite 372 beschrieben, daß Tensidmischungen in der Regel synergistische Effekte aufweisen und oft bessere Reinigungseigenschaften besitzen, als sich aus der Summe der Werte der Einzelkomponenten ergeben würde.

Waschmittel, die Alkylglykoside in Kombination mit wenigstens einem üblichen anionischen Tensid im Verhältnis 1:10 bis 10:1 enthalten, werden in der europäischen Patentanmeldung EP 070 074 beschrieben. Waschmittel, die Alkylglykoside und Aniontenside enthalten, sind auch aus der europäischen Patentanmeldung EP 092 877 bekannt. Des weiteren sind aus der europäischen Patentanmeldung EP 105 556 flüssige Waschmittel bekannt, die Alkylglykoside, bestimmte andere nichtionische Tenside und anionische Tenside enthalten. Auch aus der internationalen Patentanmeldung WO 86/02943 sind alkylglykosidhaltige Flüssigwaschmittel bekannt, die übliche Aniontenside enthalten. Aus der europäischen Patentanmeldung EP 132 043 ist ein Verfahren zur Herstellung von Alkylglykosiden unter Einsatz katalytischer Mengen eines Aniontensids in dessen Säureform bekannt. In der europäischen Patentanmeldung EP 132 046 wird vorgeschlagen, ein derartiges Herstellungsverfahren durch Zugabe bestimmter Basen nach der eigentlichen Reaktion zur Neutralisation des Katalysators zu modifizieren. Die europäische Patentanmeldung EP 0 388 810 beschreibt flüssige wäßrige Reinigungsmittel, die 3-60 Gew.-% Alkylglykosid und 0,001-0,5 Gew.-% höhere Alkohole sowie 0,001-8 Gew.-% wasserlösliches anorganisches oder organisches Salz enthalten.

Die in diesen Dokumenten erwähnten Wasch- beziehungsweise Reinigungsmittelvorprodukte auf Alkylglykosid-Basis sind relativ hochkonzentrierte wäßrige Lösungen beziehungsweise Pasten, da die für die Mischung zu fertigen Mitteln vorgesehenen Komponenten einen möglichst hohen Aktivsubstanzgehalt aufweisen sollen. Gleichzeitig müssen sie leicht handhabbar sein, das heißt, sie sollten eine möglichst niedrige Viskosität aufweisen, fließfähig und leicht pumpbar sein. Demgegenüber wird an vom Verbraucher angewendete wäßrige Flüssigprodukte, zu denen insbesondere Flüssigwaschmittel, Geschirrspülmittel und Universalreiniger, aber auch kosmetische Produkte, beispielsweise Haarshampoos oder Körperreinigungslotionen gehören, die Forderung an eine gewisse Mindestviskosität gestellt, obwohl der Aktivsubstanzgehalt derartiger Produkte in der Regel relativ niedrig ist. Aus der internationalen Patentanmeldung WO 91/04313 ist bekannt, daß die Viskosität von Mitteln, welche anionische und/oder amphotere Tenside enthalten, durch die Zugabe einer Kombination aus Alkylglykosid und Alkalichlorid reduziert wird. Dabei soll der Gehalt an Alkalichlorid so gering wie möglich gehalten werden, so daß die hauptsächliche Viskositätsreduktion auf das Alkylglykosid zurückzuführen ist.

Wäßrige Tensidzubereitungen, enthaltend 2 bis 15 Gew.-% Alkylglykosid und 0,05 bis 2 Gew.-% Aniontensid vom Sulfat- und/oder Sulfonat-Typ sind aus der deutschen Patentanmeldung DE 41 34 071 bekannt. Diese sind gelförmige, isotrope, viskoelastische Lösungen. Sie weisen eine Viskosität im Bereich von 250 mPa·s bis 10 0000 mPa·s auf und liegen somit bei für Endprodukte wünschenswerter Konzentration im für Verbraucher gut zu handhabenden Viskositätsbereich. In die aus diesem Dokument bekannten wäßrigen Tensidsysteme können jedoch nicht ohne weiteres feinteilige Feststoffe lagerstabil, das heißt ohne Phasentrennung und/oder Sedimentation, eingearbeitet werden.

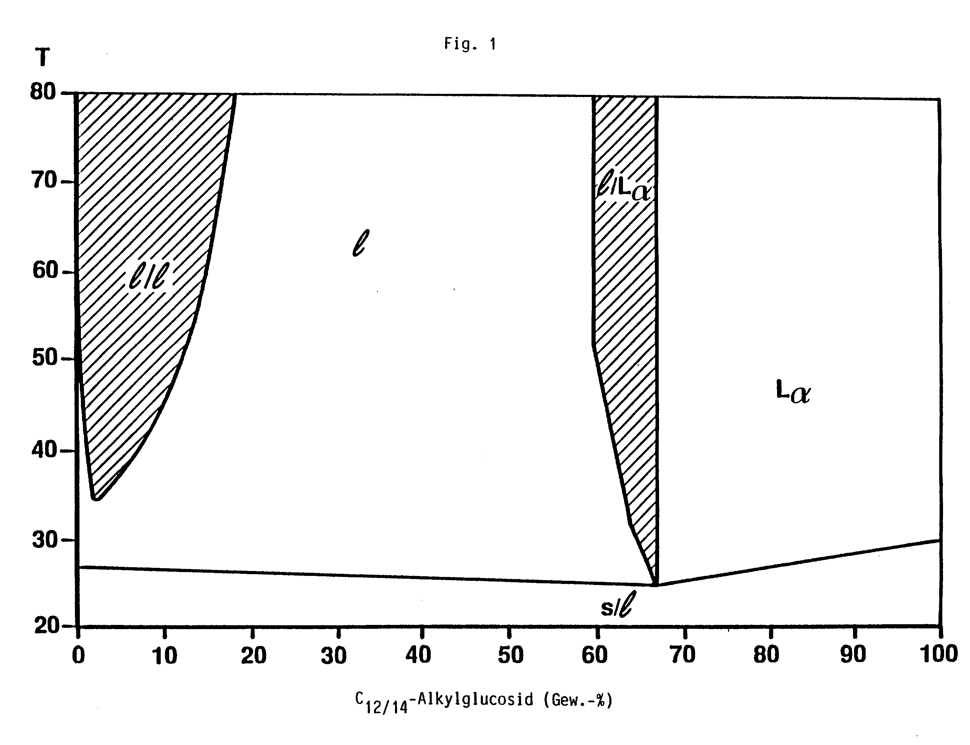

Für die Konfektionierung von Flüssigprodukten, welche ungelöste Feststoffe enthalten, sind aus Gründen der physischen Stabilität Tensidsysteme, die lamellare Flüssigkristalle ausbilden, von Interesse. Das Auftreten von flüssigkristallinen Phasen im System Wasser/Seife ist seit langer Zeit bekannt, wie beispielsweise der Übersicht von F.B. Rosevaer in J. Soc. Cosmetic Chemists 19, 1968, Seiten 581-594 zu entnehmen. In Alkylglykosid/wasser-Gemischen treten Flüssigkristalle jedoch erst bei sehr hohen, für die direkte Anwendung praktisch irrelevanten Wirkstoffkonzentrationen auf. So bildet beispielsweise ein C_{12/14}-Alkylglglucosid (Oligomerisierungsgrad 1,4) in Wasser erst bei Konzentrationen über etwa 65 Gew.-% und Temperaturen über 25 °C eine flüssigkristalline Phase (L_{α}) aus (Figur 1).

Überraschenderweise wurde nun gefunden, daß durch Zugabe geringer Mengen von mittel- bis langkettigen Alkoholen zu wäßrigen Alkylglykosidlösungen Flüssigkristalle, insbesondere lamellare Flüssigkristalle schon bei sehr niedrigen Alkylglykosidkonzentrationen auftreten. Auch wenn man diesen Systemen Aniontensid zusetzt, bricht die flüssigkristalline Phasenstruktur keineswegs zusammen. Vielmehr führt dieser Zusatz in der Regel zu einer anwendungstechnisch vorteilhaften Viskositätserhöhung und dem Entstehen einer Fließgrenze.

Gegenstand der Erfindung ist demgemäß eine wäßrige, alkylglykosid- und alkoholhaltige Tensidzubereitung, gegebenenfalls enthaltend synthetisches Aniontensid vom Sulfat-oder Sulfonat-Typ, welche dadurch gekennzeichnet ist, daß sie
0,5 Gew.-% bis 30 Gew.-% eines Alkyl- und/oder Alkenylglykosids der Formel I,

R¹-O(G)ₙ (I)

in der R¹ einen Alkyl- oder Alkenylrest mit 8 bis 22 C-Atomen, G eine Glykose-Einheit und n eine Zahl zwischen 1 und 10 bedeuten,
0,05 Gew.-% bis 12 Gew.-% eines Alkohols der Formel II,

R²-OH (II)

in der R² einen Alkyl- oder Alkenylrest mit 6 bis 20 C-Atomen und bis zu 3 C-C-Doppelbindungen bedeutet,
bis zu 30 Gew.-%, insbesondere 0,01 Gew.-% bis 28 Gew.-% eines synthetischen Aniontensids vom Sulfat- und/oder Sulfonattyp, insbesondere eines Alkyl- und/oder Alkenylsulfats der allgemeinen Formel III,

R³-OSO₃M (III)

in der R³ einen Alkyl- oder Alkenylrest mit 10 bis 22 C-Atomen und bis zu 3 C-C-Doppelbindungen und M ein Kation, insbesondere ein Alkalimetallkation, bedeuten,
sowie auf 100 Gew.-% Wasser enthält
und bei 25 °C lyotrop flüssigkristallin ist.

Ein weiterer Gegenstand der Erfindung ist ein Verfahren zur Herstellung von flüssigkristallinen wäßrigen, 0,5 Gew.-% bis 30 Gew.-% Alkyl- beziehungsweise Alkenylglykosid gemäß Formel I enthaltenden Tensidsystemen durch Mischen des Glykosids mit Wasser sowie gegebenenfalls bis zu 30 Gew.-%, bezogen auf entstehendes Tensidsystem, synthetischem Aniontensid vom Sulfat- und/oder Sulfonat-Typ und einer zur Entstehung von Flüssigkristallen bei einer Temperatur im Bereich von 20 °C bis 30 °C ausreichenden Menge Alkohol gemäß Formel II, Einwirkenlassen von starken Scherkräften zur Homogenisierung des Tensidsystems und gegebenenfalls equilibrierendes Ruhenlassen, bis sich die flüssigkristalline Phase bildet. Dies kann bei ungünstiger Wahl der Mengen der einzelnen Komponenten, insbesondere beim Einsatz längerkettiger Alkohole unter Umständen bis zu 2 Wochen benötigen, dauert in der Regel allerdings nur etwa 1,5 Stunden bis 1 Tag. Die zum Homogenisieren der wäßrigen Systeme notwendigen starken Scherkräfte können im Labormaßstab durch handelsübliche Intensivmischer, beispielsweise Ultra-Turrax^{(R)}-Rührer, eingebracht werden. Im Produktionsmaßstab können übliche Homogenisatoren bzw. Dispergatoren eingesetzt werden. Unter equilibrierendem Ruhenlassen ist ein einfaches Stehenlassen des wäßrigen Tensidsystems bei einer Temperatur im Bereich von 20 °C bis 30 °C zu verstehen. Die Reihenfolge des Zumischens der Einzelkomponenten ist beim erfindungsgemäßen Herstellungsverfahren nicht von Bedeutung. Insbesondere Wasser kann im Rahmen des Herstellungsverfahrens an jeder Stelle zugemischt werden. Vorzugsweise geht man so vor, daß man eine wäßrige Alkyl- beziehungsweise Alkenylglykosid-Lösung beziehungsweise -paste (Konzentration 30 Gew.-% bis 60 Gew.-%) vorlegt, einen Teil des Wassers zumischt, den Alkohol zusetzt und schließlich die restliche Wassermenge, in welcher das gewünschtenfalls einzuarbeitende Aniontensid enthalten sein kann, zusetzt.

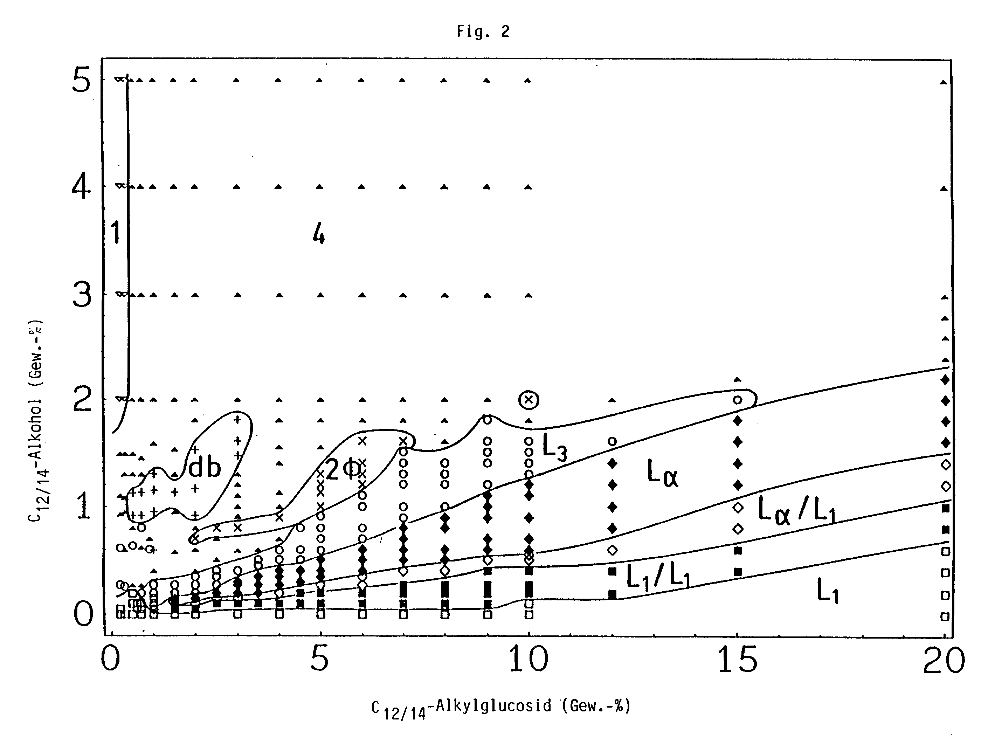

In diesem Zusammenhang ist zu bemerken, daß nach unserer Erfahrung nicht im gesamten Mehrstoffsystem aus Wasser/Alkyl- beziehungsweise Alkenylglykosid/Alkohol/gegebenenfalls Aniontensid Flüssigkristalle auftreten. Der Fachmann ist jedoch ohne weiteres in der Lage, die erfindungsgemäßen flüssigkristallinen Systeme zu erkennen, beispielsweise anhand von Texturen (zum Beispiel Bandstrukturen oder spherische Teilchenstrukturen, wie in dem obengenannten Artikel von F.B. Rosevaer abgebildet) unter einem Mikroskop mit gekreuzten Polarisatoren oder Bestimmung der Lage der Peaks und ihrer Intensität bei der Röntgenkleinwinkelstreuung. Zur Erläuterung sei auf das in Figur 2 wiedergebene Phasendiagramm des Systems C_{12/14}-Alkylglucosid/C_{12/14}-Fettalkohol/Wasser bei 25 °C hingewiesen. In dieser Figur bezeichnen "L_{α}", "db" beziehungsweise "L₃" erfindungsgemäße flüssigkristalline Phasen (lamellar flüssigkristallin, doppelbrechend beziehungsweise strömungsdoppelbrechend). Die mit "1" wie auch die mit "L₁" bezeichneten Phasen sind isotrop flüssig, in den mit "L₁/L₁" und "4" bezeichneten Gebieten liegen jeweils 2 flüssige isotrope Phasen vor. In den Gebieten "2φ" und "L_{α}/L₁" liegen jeweils eine flüssigkristalline und eine isotropflüssige Phase nebeneinander vor.

Vorzugsweise enthalten die erfindungsgemäßen flüssigkristallinen Zubereitungen 2 Gew.-% bis 20 Gew.-%, insbesondere 3 Gew.-% bis 15 Gew.-% Alkylbeziehungsweise Alkenylglykosid gemäß Formel I, 0,1 Gew.-% bis 10 Gew.-%, insbesondere 0,5 Gew.-% bis 5 Gew.-% Alkohol gemäß Formel II, 0,1 Gew.-% bis 25 Gew.-%, insbesondere 0,5 Gew.-% bis 10 Gew.-% synthetisches Aniontensid und auf 100 Gew.-% Wasser. Bevorzugt beträgt das Gewichtsverhältnis von Alkyl- beziehungsweise Alkenylglykosid zu Alkohol 50:1 bis 1:5, insbesondere 15:1 bis 1:1. Falls zusätzlich Aniontensid enthalten ist, liegen die Alkoholmengen vorzugsweise etwas höher; das Gewichtsverhältnis von Alkyl- beziehungsweise Alkenylglykosid zu Alkohol beträgt dann vorzugsweise 45:1 bis 1:4, insbesondere 10:1 bis 1:2.

Die erfindungsgemäßen flüssigkristallinen Tensidzubereitungen weisen bei 25 °C vorzugsweise Viskositäten im Bereich von 10 mPa.s bis 150 000 mPa.s, insbesondere von 80 mPa.s bis 10 000 mPa.s bei einer Scherrate von 1 s⁻¹ auf. Dabei wurde beobachtet, daß bei höheren Gehalten von insbesondere langkettigen Alkoholen mit Alkylresten im Bereich von C₁₂ bis C₁₆ besonders hohe Viskositäten auftreten. In einer bevorzugten Ausgestaltung der Erfindung enthalten die flüssigkristallinen Tensidzubereitungen daher Alkyl- beziehungsweise Alkenylglykosid und C₁₂- bis C₁₆-Alkohol in Gewichts verhältnissen von 4:1 bis 2:1 und weisen unter den genannten Bedingungen Viskositäten im Bereich von 50 000 mPa.s bis 150 000 mPa.s auf. Falls synthetisches Aniontensid enthalten ist, liegt die Viskosität unter den genannten Meßbedingungen vorzugsweise im Bereich von 80 mPa.s bis 12 000 mPa.s, insbesondere von 100 mPa.s bis 10 000 mPa.s. Die erfindungs" gemäßen flüssigkristallinen Tensidzubereitungen weisen vorzugsweise, insbesondere wenn sie aniontensidhaltig sind, eine Fließgrenze im Bereich vor 0,5 Pa bis 50 Pa bei 25 °C auf.

Die für die erfindungsgemäßen Tensidzubereitungen geeigneten Alkylglykoside und ihre Herstellung werden zum Beispiel in den europäischen Patentanmeldungen EP 92 355, EP 301 298, EP 357 969 und EP 362 671 oder der US-amerikanischen Patentschrift US 3 547 828 beschrieben. Bei den Glykosidkomponenten ((G)ₙ in Formel I) derartiger Alkylglykoside handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad (n in Formel I) kann als analytisch zu ermittelnde Größe auch nicht-ganzzahlige Zahlenwerte annehmen; er liegt in der Regel bei Werten zwischen 1 und 10, bei den vorzugsweise eingesetzten Alkylglykosiden unter einem Wert von 3, insbesondere zwischen 1,2 und 1,4. Bevorzugter Monomer-Baustein ist wegen der guten Verfügbarkeit Glucose.

Der Alkyl- beziehungsweise Alkenylteil (R¹ in Formel I) der in den erfindungsgemäßen Tensidzubereitungen enthaltenen Alkyl- beziehungsweise Alkenylglykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole und/oder Guerbet-Alkohole, zur Herstellung verwendbarer Alkylglykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten und ein- oder mehrfach ungesättigte Alkohole dieser Kettenlängen, zu denen beispielsweise Oleylalkohol, Elaidylalkohol, Linoleylalkohol, Linolenylalkohol, Gadoleylalkohol und Erucaalkohol gehören, sowie deren Gemische. Besonders geeignete Alkylglykoside enthalten einen Kokosfettalkylrest, das heißt Mischungen mit im wesentlichen R¹=Dodecyl und R¹=Tetradecyl.

Die Alkyl- beziehungsweise Alkenylglykoside können bekanntlich herstellungsbedingt geringe Mengen, normalerweise 1 bis 2 Gew.-% bezogen auf Alkyl- beziehungsweise Alkenylglykosid, an nicht umgesetztem freiem Alkohol enthalten. Diese Mengen an Alkohol reichen jedoch bei weitem nicht aus, um zu erfindungsgemäßen flüssigkristallinen Systemen zu führen

Zu den in den erfindungsgemäßen flüssigkristallinen Tensidzubereitungen enthaltenen lineraren oder verzweigtkettigen, gesättigten oder ein- bis dreifach ungesättigten Alkoholen gemäß Formel II gehören neben den obengenannten Alkoholen, die Ausgangsstoffe für die Alkyl- beziehungsweise Alkenylglykoside sein können, auch kürzerkettige Alkohole, wobei jedoch Alkohole mit weniger als 6 C-Atomen erfindungsgemäß nicht brauchbar sind. Insbesondere werden die Alkohole aus Hexanol, Heptanol, Octanol, Decanol, Undecanol, 10-Undecenol, Dodecanol, Tridecanol, Tetradecanol, Pentadecanol, Hexadecanol, Heptadecanol, Octadecanol, Oleylalkohol, Elaidylalkohol, Linoleylalkohol und/oder Linolenylalkohol ausgewählt, wobei deren lineare Isomere mit primärer Alkoholgruppe bevorzugt sind. Dabei muß die Kettenlänge des Alkyl- beziehungsweise Alkenylteils des in der erfindungsgemäßen flüssigkristallinen Zubereitung enthaltenen Glykosids keineswegs mit derjenigen des Alkohols übereinstimmen.

Zu den in den erfindungsgemäßen Tensidzubereitungen gegebenenfalls enthaltenen Aniontensiden vom Sulfat- oder Sulfonat-Typ gehören insbesondere Alkylsulfate, Alkansulfonate, Alkylbenzolsulfonate, α-Sulfofettsäuren, deren Carbonsäureester, Sulfonierungsprodukte von Alkenen oder ungesättigten Fettsäuren sowie deren Gemische.

Die im Rahmen der Erfindung einsetzbaren Alkylsulfate gemäß Formel III sind bekannte anionische Tenside, die in der Regel durch Umsetzung von aliphatischen primären Alkoholen mit einem Sulfatierungsreagenz, beispielsweise Schwefeltrioxid oder Chlorsulfonsäure sowie nachfolgender Neutralisation und Hydrolyse der entstandenen Reaktionsprodukte, vorzugsweise mit Alkali-, Ammonium- oder Alkyl- beziehungsweise Hydroxyalkyl-substituierten Ammoniumbasen, hergestellt werden. Alkylsulfate, auf die sich die Erfindung erstreckt, leiten sich vorzugsweise von Fettalkoholen mit 12 bis 22 Kohlenstoffatomen, insbesondere 12 bis 18 Kohlenstoffatomen, ab. Typische Beispiele hierfür sind Laurylalkohol, Myristylalkohol, Cetylalkohol, Stearylalkohol oder Behenylalkohol. Die Alkylsulfate können sich auch von technischen Alkoholgemischen ableiten, wie sie zum Beispiel bei der Hydrierung von technischen Fettsäureestergemischen natürlicher Herkunft oder von Aldehyden aus der Roelen'schen Oxosynthese anfallen. Bevorzugt sind hierbei Alkylsulfate auf der Basis von technischen Kokos- oder Talgalkoholschnitten.

Derartige Alkylsulfate können auch, bedingt durch den Anteil ungesättigter Alkohole im Einsatzmaterial für die Sulfatierung, mehr oder weniger große Mengen an Sulfatierungsprodukten von ein-, zwei- oder dreifach ungesättigten Alkoholen der genannten Kettenlängen enthalten. Dabei handelt es sich, da bei Einsatz ungesättigter Alkohole bei der Sulfatierung auch eine Anlagerung des Sulfatierungsmittels an die Doppelbindung stattfinden kann, in der Regel um Mischungen aus Alkenylsulfaten mit Stoffen, die eine innenständige Sulfonatgruppe beziehungsweise eine Sulfonat- und eine Sulfatgruppe enthalten. Der Begriff "Alkylsulfat" umfaßt daher im Rahmen der vorliegenden Erfindung auch derartige Gemische. Vorzugsweise sind aus ungesättigten Alkoholen hervorgegangene Sulfierungsprodukte, wenn im Alkylsulfat vorhanden, in Mengen nicht über 80 Gew.-%, insbesondere von 30 Gew.-% bis 70 Gew.-%, im eingesetzten Alkylsulfat, bezogen auf gesamte in diesem enthaltene aniontensidische Aktivsubstanz, enthalten.

Die für die Einarbeitung in die erfindungsgemäßen Zubereitungen geeigneten Sulfofettsäuresalze sind neutralisierte Derivate der mindestens eine Doppelbindung enthaltenden Fettsäuren mit 8 bis 22 C-Atomen. Zu diesen gehören insbesondere die Sulfonierungsprodukte von Lauroleinsäure, Myristoleinsäure, Palmitoleinsäure, Ölsäure, Gadoleinsäure und Erucasäure. Derartige Sulfofettsäuresalze werden durch Umsetzung der ungesättigten Fettsäuren mit einem Sulfonierungsmittel und anschließende Neutralisation und Hydrolyse mit den genannten wäßrigen Basen nach bekannten Verfahren, wie.sie zum Beispiel in der britischen Patentschrift GB 1 278 421 oder der internationalen Patentanmeldung WO 90/06300 beschrieben sind, hergestellt. Dabei entstehen Gemische der eine Sulfo- und eine Hydroxygruppe tragenden gesättigten Fettsäuren mit durch formale Eliminierung eines Molequivalents Wasser aus diesen entstehenden ungesättigten, eine Sulfogruppe tragenden Fettsäuren. Durch analoge Reaktion können aus Alkenen mit vorzugsweise 12 bis 22 C-Atomen oberflächenaktive Gemische aus Hydroxyalkylsulfonaten mit Alkensulfonaten erzeugt werden.

α-Sulfofettsäuresalze können durch Umsetzung von Fettsäuren, die 6 bis 22, vorzugsweise 16 bis 18 Kohlenstoffatome enthalten und vorzugsweise eine Iodzahl kleiner 20 aufweisen, mit einem Sulfiermittel und nachfolgende Neutralisation mit wäßrigen Basen hergestellt werden. Als Sulfiermittel kommen in diesem Fall insbesondere Schwefelsäure, Oleum, Chlorsulfonsäure oder gasförmiges Schwefeltrioxid im Gemisch mit einem Inertgas in Betracht. Als Neutralisationsbasen eignen sich vor allem wäßrige Lösungen von Alkali- und Erdalkalihydroxiden oder Ammoniak. Alternativ können auch die Ester von Fettsäuren der genannten Art mit C₁- bis C₄-Alkoholen, insbesondere Fettsäuremethylester, sulfoniert werden, wobei die erfindungsgemäß brauchbaren α -Sulfofettsäureester entstehen. Durch Verwendung von wäßriger Base im Überschuß und unter Abspaltung von Methanol können sie zu den entsprechenden α-Sulfofettsäuresalzen verseift und neutralisiert werden. Typische Beispiele für Fettsäuren der genannten Art sind Capronsäure, Caprylsäure, Caprinsäure, Laurinsäure, Myristinsäure oder Behensäure. Substanzen mit besonders günstigen Detergenseigenschaften werden auf Basis von Palmitin- und Stearinsäure erhalten, die aus diesen Gründen bevorzugt sind. Diese Fettsäuren können auch in Form technischer Gemische vorliegen, wie sie üblicherweise bei der Spaltung pflanzlicher oder tierischer Fettsäureglycerinester anfallen. Weisen derartige Gemische hohe Anteile ungesättigter Fettsäuren auf, können sie in an sich bekannter Weise durch Hydrierung z. B. in Gegenwart von Nickelkatalysatoren in Gemische weitgehend gesättigter Fettsäuren überführt werden, deren Iodzahl kleiner 20 ist.

Bei den erfindungsgemäß einsetzbaren Alkansulfonaten handelt es sich um Substanzen, die durch Sulfoxidation von Kohlenwasserstoffen, welche vorzugsweise 10 bis 20 C-Atome enthalten, gewonnen werden. Dabei entstehen in der Regel Produkte mit statistischer Verteilung der Sulfonsäure-Substituenten, die gewünschtenfalls in bekannter Weise getrennt werden können. Für die erfindungsgemäßen Mischungen sind sekundäre Alkansulfonate mit 12 bis 17 C-Atomen besonders geeignet. Als Kationen kommen auch in diesem Fall insbesondere solche aus der Gruppe der Alkaliionen, Ammonium- oder Alkylbeziehungsweise Hydroxyalkyl-substituierten Ammoniumionen in Betracht.

Unter den genannten synthetischen Aniontensiden sind Alkylsulfate, Alkansulfonate und/oder Sulfofettsäureester bzw. Salze besonders bevorzugt.

In einer bevorzugten Ausführungsform enthält eine erfindungsgemäße Tensidzubereitung 3 Gew.-% bis 10 Gew.-% Alkyl- beziehungsweise Alkenylglykosid gemäß Formel I, 0,75 Gew.-% bis 7,5 Gew.-% Alkohol gemäß Formel II, insbesondere mit 6 bis 18 C-Atomen, bis zu 20 Gew.-%, insbesondere 1 Gew.-% bis 15 Gew.-% synthetisches Aniontensid, insbesondere vom Sulfat-Typ, und als Rest auf 100 Gew.-% Wasser.

Die erfindungsgemäßen flüssigkristallinen Tensidzubereitungen weisen ausgezeichnete Reinigungseigenschaften und eine hohe Kaltwasserlöslichkeit auf. Sie werden daher vorzugsweise als Wasch,- Spül- oder Reinigungsmittel sowie insbesondere in der Haar- und Körperpflege verwendet oder dienen als lagerstabile, leicht handhabbare Vorgemische zur Herstellung solcher Mittel. Im Rahmen der Herstellung solcher Endprodukte ist die Zugabe anderer in derartigen Mitteln üblicher Bestandteile, zu denen insbesondere Buildersubstanzen, wie Alkalitripolyphosphate, -citrate, Zeolithe und Schichtsilikate, Korrosionsinhibitoren, Bleichmittel, Bleichaktivatoren, optische Aufheller, Enzyme, Vergrauungsinhibitoren, antimikrobielle Wirkstoffe, Abrasivstoffe, beispielsweise Alkalibicarbonate oder -tetraborate, Schaumregulatoren bzw. -stabilisatoren, Konservierungsmittel, pH-Regulatoren, Trübungs- und Perlglanzmittel, Farb- und Duftstoffe sowie zusätzliche Tenside gehören, zu den erfindungsgemäßen Zubereitungen möglich. Von besonderem Vorteil sind die erfindungsgemäßen flüssigkristallinen Tensidzubereitungen, wenn zur Herstellung der genannten Mittel feinteilige Feststoffe eingearbeitet werden sollen, beispielsweise wasserunlösliche Buildersubstanzen, insbesondere Zeolith Na-A in Waschmittelqualität, und/ oder Abrasivstoffe, beispielsweise Quarzmehl, Marmormehl oder feinteiliges Aluminiumoxid, da diese durch die erfindungsgemäßen flüssigkristallinen Systeme sehr effektiv stabilisiert, das heißt in homogener Verteilung in der Flüssigkeit gehalten, werden. Aus diesem Grund ist die Verwendung der erfindungsgemäßen flüssigkristallinen Tensidzubereitungen zur Stabilisierung von feinteiligen wasserunlöslichen Bestandteilen flüssiger Wasch-, Spül-, Reinigungs- oder kosmetischer Mittel ein bevorzugtes Einsatzgebiet der erfindungsgemäßen Zubereitungen. Dabei war überraschenend, daß die erfindungsgemäßen flüssigkristallinen Tensidzubereitungen in der Lage sind, sehr große Mengen derartiger wasserunlöslicher Bestandteile in homogener Verteilung zu stabilisieren. Vorzugsweise werden die erfindungsgemäßen flüssigkristallinen Tensidzubereitungen daher in Mitteln, insbesondere Reinigungsmitteln, mit hohen Anteilen, insbesondere 10 Gew.-% bis 60 Gew.-% und besonders bevorzugt 20 Gew.-% bis 50 Gew.-% an wasserunlöslichen Bestandteilen, insbesondere Abrasivstoffen, verwendet. Die zum Stabilisieren notwendige Menge an erfindungsgemäßem Tensidsystem ist relativ klein und wird vorzugsweise so gewählt, daß im fertigen Mittel das Gewichtsverhältnis von feinteiligem wasserunlöslichem Bestandteil zu der Summe aus Alkyl- und/oder Alkenylglykosid der Formel I und Alkohol der Formel II im Bereich von 20 : 1 bis 1 : 1, insbesondere von 15 : 1 bis 2 : 1 liegt.

### Beispiele

### Beispiel 1

Durch Vermischen der Einzelkomponenten mit Hilfe eines Ultra-Turrax^{(R)}-Rührers wurden wäßrige Mischungen **M1** bis **M3** aus den in der nachfolgenden Tabelle 1 angegebenen Komponenten in den angebenen Mengenverhältnissen (Gew.-%) hergestellt. Sämtliche der aufgeführten Tensidzubereitungen waren flüssigkristallin und besaßen bei 25 °C die angebene Viskosität (gemessen mit einem schubspannungskontrollierten Rotationsrheometer Carri-med^{(R)} CS 100 bei einer Scherrate D von 1 s⁻¹).

**Tabelle 1:**

| Viskositäten erfindungsgemäßer Zubereitungen | | | |
|---|---|---|---|
| | **M1** | **M2** | **M3** |
| C_{12/14}-Alkylglucosid^{a)} | 10 | 10 | 10 |
| C_{12/14}-Alkohol^{b)} | 1 | 1,5 | 1,75 |
| Wasser | 89 | 88,5 | 88,25 |
| Viskosität [mPa.s] | 180 | 6000 | 1000 |

| | | | |
|---|---|---|---|
| a) Oligomerisierungsgrad 1,4 | | | |
| b) Lorol^{(R)} spezial, Hersteller Henkel | | | |

### Beispiel 2

Wie in Beispiel 1 beschrieben, wurde aus C_{12/16}-Alkylglucosid (Oligomerisierungsgrad 1,4) und C_{12/14}-Alkohol eine flüssigkristalline wäßrige Formulierung hergestellt. Dieser wurde Marmormehl zugemischt, so daß man ein flüssiges Reinigungsmittel **M7** der in der nachfolgenden Tabelle 2 angegebenen Zusammensetzung erhielt, das bei Lagerung über mehrere Wochen seine Homogenität nicht verlor und die in Tabelle 3 angegebenen Viskositätseigenschaften, bestimmt mit einem schergeschwindigkeitskontrollierten Rotationsrheometer Rheometrics^{(R)} RFS II mit Platte-Platte-Meßsystem (2 cm Durchmesser, 2 mm Spalt), besaß.

**Tabelle 2:**

| Zusammensetzung [Gew.-%] | |
|---|---|
| | **M7** |
| C_{12/16}-Alkylglucosid | 5 |
| C_{12/14}-Alkohol | 1,5 |
| Marmormehl | 45 |
| Wasser | 48,5 |

**Tabelle 3:**

| Viskoelastische Eigenschaften von **M7** | |
|---|---|
| Viskosität [mPa.s] bei 30 s⁻¹ und 10 °C | 1270 |
| Nullscherviskosität [mPa.s] bei 10 °C | 430000 |
| Viskosität [mPa.s] bei 30 s⁻¹ und 20 °C | 320 |
| Nullscherviskosität [mPa.s] bei 20 °C | 330000 |
| Fließgrenze [Pa] bei 20 °C | 3,0 |
| Viskosität [mPa.s] bei 30 s⁻¹ und 45 °C | 270 |
| Nullscherviskosität [mPa.s] bei 45 °C | 5750000 |
| Fließgrenze [Pa] bei 45 °C | 0,3 |

### Bezugszeichenliste

### Fig. 1

- T :: Temperatur [°C]
- *l* :: 1 isotrope flüssige Phase
- L_{α} :: lamellar-flüssigkristalline Phase
- *l/l* :: Zweiphasengebiet (2 isotrope flüssige Phasen)
- s*/l* :: Zweiphasengebiet (Feststoff und isotrope Flüssigkeit)
- *l/*L_{α} :: Zweiphasengebiet (lamellar-flüssigkristalline Phase und isotrope flüssige Phase)

### Fig. 2

Erläuterungen zu Abkürzungen in den Phasendiagrammen
- 1 :: isotrop, flüssig
- 4 :: 2 flüssige Phasen
- db :: doppelbrechende Phase
- L₃:: strömungsdoppelbrechende Phase
- L_{α} :: lamellare, flüssigkristalline Phase
- 2φ :: Zweiphasengebiet (isotrop flüssig / L₃)
- L_{α}/L₁:: Zweiphasengebiet (flüssigkristallin / isotrop)
- L₁ :: isotrop flüssig (mizellare Phase)
- L₁/L₁:: 2 flüssige Phasen

## Patentansprüche

1. Wäßrige, alkylglykosid- und alkoholhaltige Tensidzubereitung, gegebenenfalls enthaltend synthetisches Aniontensid vom Sulfat- oder Sulfonat-Typ, dadurch gekennzeichnet, daß sie 0,5 Gew.-% bis 30 Gew.-% eines Alkyl- und/oder Alkenylglykosids der Formel I,
R¹-O(G)ₙ (I)
in der R¹ einen Alkyl- oder Alkenylrest mit 8 bis 22 C-Atomen, G eine Glykose-Einheit und n eine Zahl zwischen 1 und 10 bedeuten,
0,05 Gew.-% bis 12 Gew.-% eines Alkohols der Formel II,
R²-OH (II)
in der R² einen Alkyl- oder Alkenylrest mit 6 bis 20 C-Atomen und bis zu 3 C-C-Doppelbindungen bedeutet,
bis zu 30 Gew.-% eines synthetischen Aniontensids vom Sulfat- und/oder Sulfonattyp
sowie auf 100 Gew.-% Wasser enthält
und bei 25 °C lyotrop flüssigkristallin ist.

2. Tensidzubereitung nach Anspruch 1, dadurch gekennzeichnet, daß das Gewichtsverhältnis von Alkylglykosid gemäß Formel I zu Alkohol gemäß Formel II 50:1 bis 1:5, insbesondere 15:1 bis 1:1 beträgt.

3. Tensidzubereitung nach Anspruch 1 oder 2, dadurch gekennzeichnet, daß der Alkyl- beziehungsweise Alkenylrest R¹ in Formel I 8 bis 16 C-Atome aufweist.

4. Tensidzubereitung nach einem der Ansprüche 1 bis 3, dadurch gekennzeichnet, daß sie 0,01 Gew.-% bis 28 Gew.-% synthetisches Aniontensid enthält.

5. Tensidzubereitung nach einem der Ansprüche 1 bis 5, dadurch gekennzeichnet, daß das Aniontensid ein Alkyl- und/oder Alkenylsulfat der allgemeinen Formel III,
R²-OSO₃M (III)
in der R³ einen Alkyl- oder Alkenylrest mit 10 bis 22 C-Atomen und bis zu 3 C-C-Doppelbindungen und M ein Kation, insbesondere ein Alkalimetallkation, bedeuten, ist.

6. Tensidzubereitung nach Anspruch 4 oder 5, dadurch gekennzeichnet, daß das Gewichtsverhältnis von Alkylglykosid gemäß Formel I zu Alkohol gemäß Formel II 45:1 bis 1:4, insbesondere 10:1 bis 1:2 beträgt.

7. Tensidzubereitung nach einem der Ansprüche 1 bis 6, dadurch gekennzeichnet, daß sie eine Viskosität, gemessen bei einer Temperatur von 25 °C bei einer Scherrate von 1 s⁻¹ im Bereich von 10 mPa·s bis 150 000 mPa·s, insbesondere von 80 mPa·s bis 10 000 mPa·s aufweist.

8. Verfahren zur Herstellung von flüssigkristallinen wäßrigen, 0,5 Gew.-% bis 30 Gew.-% Alkyl- beziehungsweise Alkenylglykosid gemäß Formel I enthaltenden Tensidsystemen durch Mischen des Glykosids mit Wasser sowie gegebenenfalls bis zu 30 Gew.-% bezogen auf entstehendes Tensidsystem synthetischem Aniontensid vom Sulfat- und/oder Sulfonat-Typ und einer zur Entstehung von Flüssigkristallen bei einer Temperatur im Bereich von 20 °C bis 30 °C ausreichenden Menge Alkohol gemäß Formel II, Einwirkenlassen von Scherkräften und gegebenenfalls equilibrierendes Ruhenlassen, bis sich die flüssigkristalline Phase bildet.

9. Verwendung einer Tensidzubereitung gemäß einem der Ansprüche 1 bis 7 als Wasch,- Spül-, Reinigungs-, Haar- oder Körperpflegemittel.

10. Verwendung einer Tensidzubereitung gemäß einem der Ansprüche 1 bis 7 als lagerstabiles, leicht handhabbares Vorgemisch zur Herstellung von Wasch,- Spül-, Reinigungs-, Haar- oder Körperpflegemitteln.

11. Verwendung einer Tensidzubereitung gemäß einem der Ansprüche 1 bis 7 zur Stabilisierung von feinteiligen wasserunlöslichen Bestandteilen flüssiger Wasch-, Spül-, Reinigungs-, Haar- oder Körperpflegemittel.

12. Verwendung nach Anspruch 11, dadurch gekennzeichnet, daß das Mittel 10 Gew.-% bis 60 Gew.-%, insbesondere 20 Gew.-% bis 50 Gew.-% feinteiligen wasserunlöslichen Bestandteil enthält.

13. Verwendung nach einem der Ansprüche 11 oder 12, dadurch gekennzeichnet, daß das Gewichtsverhältnis von feinteiligem wasserunlöslichem Bestandteil zu der Summe aus Alkyl- und/oder Alkenylglykosid der Formel I und Alkohol der Formel II im Bereich von 20 : 1 bis 1 : 1, insbesondere von 15 : 1 bis 2 : 1 liegt.

## Claims

1. An aqueous alkyl-glycoside- and alcohol-containing surfactant preparation optionally containing synthetic anionic surfactant of the sulfate or sulfonate type, characterized in that it contains
0.5% by weight to 30% by weight of an alkyl and/or alkenyl glycoside corresponding to formula I:
R¹-O(G)ₙ (I)
where R¹ is an alkyl or alkenyl radical containing 8 to 22 carbon atoms, G is a glycose unit and n is a number of 1 to 10,
0.05% by weight to 12% by weight of an alcohol corresponding to formula II:
R²-OH (II)
where R² is an alkyl or alkenyl radical containing 6 to 20 carbon atoms and up to 3 C-C double bonds,
up to 30% by weight of a synthetic anionic surfactant of the sulfate and/or sulfonate type and
water to 100% by weight
and shows lyotropic liquid crystalline behavior at 25°C.

2. A surfactant preparation as claimed in claim 1, characterized in that the ratio by weight of alkyl glycoside corresponding to formula I to alcohol corresponding to formula II is 50:1 to 1:5 and, more particularly, 15:1 to 1:1.

3. A surfactant preparation as claimed in claim 1 or 2, characterized in that the alkyl or alkenyl radical R¹ in formula I contains 8 to 16 carbon atoms.

4. A surfactant preparation as claimed in any of claims 1 to 3, characterized in that it contains 0.01% by weight to 28% by weight of synthetic anionic surfactant.

5. A surfactant preparation as claimed in any of claims 1 to 5, characterized in that the anionic surfactant is an alkyl and/or alkenyl sulfate corresponding to general formula III:
R³-OSO₃M (III)
in which R³ is an alkyl or alkenyl radical containing 10 to 22 carbon atoms and up to 3 C-C double bonds and M is a cation, more particularly an alkali metal cation.

6. A surfactant preparation as claimed in claim 4 or 5, characterized in that the ratio by weight of alkyl glycoside corresponding to formula I to alcohol corresponding to formula II is 45:1 to 1:4 and, more particularly, 10:1 to 1:2.

7. A surfactant preparation as claimed in any of claims 1 to 6, characterized in that it has a viscosity, as measured at a temperature of 25°C and at a shear rate of 1 s⁻¹, in the range from 10 mPa·s to 150,000 mPa·s and more particularly in the range from 80 mPa·s to 10,000 mPa·s.

8. A process for the production of liquid crystalline, aqueous surfactant systems containing 0.5% by weight to 30% by weight of alkyl or alkenyl glycoside corresponding to formula I by mixing the glycoside with water and optionally up to 30% by weight, based on the surfactant system formed, of synthetic anionic surfactant of the sulfate and/or sulfonate type and alcohol corresponding to formula II in a quantity sufficient for the formation of liquid crystals at a temperature of 20°C to 30°C, exposing the mixture to shear forces and optionally leaving it standing to equilibrate until the liquid crystalline phase has formed.

9. The use of the surfactant preparation claimed in any of claims 1 to 7 as a laundry detergent, dishwashing detergent, cleaning product, hair-care or body-care formulation.

10. The use of the surfactant preparation claimed in any of claims 1 to 7 as a storable, easy-to-handle compound for the production of laundry detergents, dishwashing detergents, cleaning products, hair-care or body-care formulations.

11. The use of the surfactant preparation claimed in any of claims 1 to 7 for stabilizing fine-particle water-insoluble constituents of liquid laundry detergents, dishwashing detergents, cleaning products, hair-care or body-care formulations.

12. The use claimed in claim 11, characterized in that the formulation contains 10% by weight to 60% by weight and, more particularly, 20% by weight to 50% by weight of a fine-particle water-insoluble component.

13. The use claimed in claim 11 or 12, characterized in that the ratio by weight of fine-particle water-insoluble component to the sum of alkyl and/or alkenyl glycoside corresponding to formula I and alcohol corresponding to formula II is in the range from 20:1 to 1:1 and more particularly in the range from 15:1 to 2:1.

## Revendications

1. Composition aqueuse de tensioactifs contenant des alkylglycosides et de l'alcool, le cas échéant contenant un tensioactif anionique synthétique de type sulfate ou sulfonate
caractérisée en ce qu'
elle contient 0,5 % en poids à 30 % en poids d'un glycoside d'alkyle et/ou d'alkényle selon la formule I
R¹-O(G)ₙ (I)
dans laquelle R¹ représente un radical alkyle ou alkényle comportant 8 à 22 atomes de carbone, G représente une unité de glycose et n est un nombre compris entre 1 et 10, 0,05 % en poids à 12 % en poids d'un alcool selon la formule II,
R²-OH (II)
dans laquelle R² représente un radical alkyle ou alkényle comportant 6 à 20 atomes de carbone et jusqu'à 3 doubles liaisons C-C, jusqu'à 30 % en poids d'un tensioactif anionique synthétique de type sulfate et/ou sulfonate ainsi que de l'eau sur 100 % et qui est un cristal liquide lyotrope à 25°C.

2. Composition de tensioactifs selon la revendication 1,
caractérisée en ce que
le rapport en poids entre le glycoside d'alkyle selon la formule I et l'alcool selon la formule II est compris entre 50:1 et 1:5, en particulier entre 15:1 et 1:1.

3. Composition de tensioactifs selon la revendication 1 ou 2,
caractérisée en ce que
le radical alkyle ou alkényle R¹ dans la formule I comporte 8 à 16 atomes de carbone.

4. Composition de tensioactifs selon une des revendications 1 à 3,
caractérisée en ce qu'
elle contient 0,01 % en poids à 28 % en poids d'un tensioactif anionique synthétique.

5. Composition de tensioactifs selon une des revendications 1 à 4,
caractérisée en ce que
le tensioactif anionique est un sulfate d'alkyle ou d'alkényle selon la formule générale III,
R²-OSO₃M (III)
dans laquelle R³ représente un radical alkyle ou alkényle comportant 10 à 22 atomes de carbone et jusqu'à 3 doubles liaisons et M représente un cation, en particulier un cation de métal alcalin.

6. Composition de tensioactifs selon la revendication 4 ou 5,
caractérisée en ce que
le rapport en poids entre le glycoside d'alkyle selon la formule I et l'alcool selon la formule II est compris entre 45:1 et 1:4, en particulier entre 10:1 et 1:2.

7. Composition de tensioactifs selon une des revendications 1 à 6,
caractérisée en ce qu'
elle présente une viscosité comprise entre 10 mPa.s et 150 000 mPa.s, en particulier entre 80 mPa.s et 10 000 mPa.s, mesurée à une température de 25°C à une fréquence de cisaillement de 1 s⁻¹.

8. Procédé pour la fabrication de systèmes de tensioactifs aqueux mésomorphes, contenant 0,5 % en poids à 30 % en poids de glycoside d'alkyle ou d'alkényle selon la formule I, en ce que l'on mélange le glycoside avec de l'eau et le cas échéant jusqu'à 30 % de tensioactif anionique synthétique du type sulfate ou sulfonate par rapport au système tensioactif formé, et une quantité d'alcool selon la formule II suffisante pour la formation de cristaux liquides à une température comprise entre 20°C et 30°C, que l'on laisse agir des forces de cisaillement et que l'on laisse le cas échéant reposer jusqu'à équilibre jusqu'à ce que la phase cristal liquide se forme.

9. Utilisation d'une composition de tensioactif selon une des revendications 1 à 7 comme produit de lavage, de lessive, de nettoyage, shampooing ou d'entretien du corps.

10. Utilisation d'une composition de tensioactif selon une des revendications 1 à 7 comme prémélange stable au stockage et facilement manipulable pour la fabrication de produits de lavage, de vaisselle, de nettoyage ou d'entretien des cheveux ou du corps.

11. Utilisation d'une composition de tensioactif selon une des revendications 1 à 7 pour la stabilisation de constituants finement divisés insolubles dans l'eau de produits liquides de lavage, de vaisselle, de nettoyage ou d'entretien des cheveux ou du corps.

12. Utilisation selon la revendication 11,
caractérisée en ce que
le produit contient 10 % en poids à 60 % en poids, en particulier 20 % en poids à 50 % en poids de constituant finement divisé insoluble dans l'eau.

13. Utilisation selon la revendication 11 ou 12,
caractérisée en ce que
le rapport en poids entre le constituant non soluble dans l'eau finement divisé et l'ensemble des glycosides d'alkyle et/ou d'alkylène selon la formule I et l'alcool selon la formule II est compris entre 20:1 et 1:1, en particulier entre 15:1 et 2:1.
